Europäisches Patentamt

European Patent Office

Office européen des brevets

(11) Veröffentlichungsnummer: **0 464 325 B1**

(12) **EUROPÄISCHE PATENTSCHRIFT**

(45) Veröffentlichungstag der Patentschrift: **23.08.95**

(21) Anmeldenummer: **91105851.9**

(22) Anmeldetag: **12.04.91**

(51) Int. Cl.6: **C12P 13/10**, //C12N9/78

(54) **Verfahren zur Herstellung von Salzen des L-Ornithins.**

(30) Priorität: **02.07.90 DE 4020980**

(43) Veröffentlichungstag der Anmeldung:
**08.01.92 Patentblatt 92/02**

(45) Bekanntmachung des Hinweises auf die
Patenterteilung:
**23.08.95 Patentblatt 95/34**

(84) Benannte Vertragsstaaten:
**AT BE CH DE DK ES FR GB GR IT LI NL SE**

(56) Entgegenhaltungen:
**EP-A- 264 984**
**US-A- 2 988 489**

**PATENT ABSTRACTS OF JAPAN, vol. 12, no.
186 (C-500)(3033), 31 Mai 1988/**

(73) Patentinhaber: **Degussa Aktiengesellschaft
Weissfrauenstrasse 9
D-60311 Frankfurt (DE)**

(72) Erfinder: **Makryaleas, Kyriakos, Dr.
Am Rain 2
W-6463 Freigericht 1 (DE)**
Erfinder: **Drauz, Karlheinz, Dr.
Zur Marienruhe 13
W-6463 Freigericht 1 (DE)**

**Beschreibung**

Gegenstand der Erfindung ist ein Verfahren zur Herstellung von Salzen des L-Ornithins durch enzymatische Umwandlung von Arginin in L-Ornithin in Gegenwart des Enzyms L-Arginase (E.C.3.5.3.1.) in einem wäßrigen Medium und anschließende Salzbildung.

Salze des L-Ornithins sind wertvolle pharmazeutische Produkte, deren Einsatzgebiete beispielsweise die parenterale Ernährung (L-Ornithin-Acetat oder L-Ornithin-Monohydrochlorid) und die Behandlung hepatischer Erkrankungen (L-Ornithin-Aspartat oder L-Ornithin-2-Ketoglutarat) sind.

Das erfindungsgemäße Verfahren ist dadurch gekennzeichnet, daß man den für die enzymatische Umwandlung erforderlichen pH-Wert im Bereich von 8,0 bis 10,0 mit der Säure einstellt, deren Salz hergestellt werden soll, nach beendeter enzymatischer Umwandlung das Reaktionsgemisch mit der gleichen Säure auf einen pH-Wert im Bereich von 6,5 bis 7,0 einstellt und das gebildete Salz direkt aus dem Reaktionsgemisch isoliert.

Besonders vorteilhaft ist es, wenn man vor der Isolierung des gebildeten Salzes das Enzym durch Ultrafiltration aus dem Reaktionsgemisch abtrennt.

Bei der praktischen Durchführung des erfindungsgemäßen Verfahrens wird zunächst der ursprüngliche pH-Wert der wäßrigen Argininlösung von etwa 11,0 mit der Säure, deren Salz hergestellt werden soll, auf einen pH-Wert im Bereich von 8,0 bis 10,0 eingestellt. Zweckmäßigerweise wird dann eine geringe Menge eines Salzes eines zweiwertigen Metalles zwecks Erzielung einer hohen Aktivität des Enzyms zugesetzt. Besonders geeignet ist ein Zusatz von $Mn^{2+}$-Salzen in einer $10^{-3}$- bis $10^{-5}$- molaren Konzentration. Nach Zugabe der L-Arginase wird die enzymatische Umwandlung bei einer Temperatur zwischen 5°C und 50°C, vorzugsweise zwischen 20°C und 35°C vorgenommen. Die erforderliche Reaktionszeit hängt von der eingesetzten Enzymmenge ab und liegt im allgemeinen zwischen 5 und 48 Stunden.

Das Arginin wird zweckmäßig in einer Konzentration von 5 bis 40 Gewichtsprozent eingesetzt, und zwar vorzugsweise in der L-Form. Es kann zwar auch in der D,L-Form eingesetzt werden, aber dann muß nach beendeter enzymatischer Umwandlung und vor der Neutralisation des Reaktionsgemisches das nicht umgesetzte D-Arginin vom gebildeten L-Ornithin getrennt werden, was zum Beispiel durch Ionenaustauschchromatographie geschehen kann.

Die L-Arginase wird aus tierischer Leber gewonnen und kann sowohl in nativer Form als auch in geeignet stabilisierter Form eingesetzt werden. Sie ist in beiden Formen im Handel erhältlich.

Nach beendeter enzymatischer Umwandlung und gegebenenfalls nach Abtrennung des nicht umgesetzten D-Arginins wird das Reaktionsgemisch mit der Säure, deren Salz hergestellt werden soll, auf einen pH-Wert im Bereich von 6,5 bis 7,0 eingestellt, d.h. neutralisiert.

Das gebildete Salz des L-Ornithins kann dann direkt aus dem Reaktionsgemisch isoliert werden, zum Beispiel durch Einengen bis zur Kristallisation oder durch Ausfällen mit einem mit Wasser mischbaren organischen Lösungsmittel, vorzugsweise mit Methanol oder insbesondere Ethanol.

Durch das erfindungsgemäße Verfahren können praktisch beliebige Salze des L-Ornithins hergestellt werden. Es können dies die Salze anorganischer Säuren, wie Salzsäure, Schwefelsäure oder Phosphorsäure sein. Besonders geeignet ist das erfindungsgemäße Verfahren aber zur Herstellung der Salze des L-Ornithins mit organischen Säuren. Geeignete organische Säuren sind beispielsweise gesättigte aliphatische Monocarbonsäuren, wie Ameisensäure, Essigsäure oder Propionsäure; ungesättigte aliphatische Monocarbonsäuren, wie Ölsäure, Linolsäure oder Linolensäure; funktionalisierte Säuren, wie Hydroxycarbonsäuren (z.B. Milchsäure oder Mandelsäure), Ketocarbonsäuren (z.B. $\alpha$-Ketoglutarsäure), Aminocarbonsäuren (z.B. Asparginsäure, Glutaminsäure oder Pyroglutaminsäure); gesättigte aliphatische Dicarbonsäuren, wie Bernsteinsäure oder Adipinsäure; ungesättigte aliphatische Dicarbonsäuren, wie Maleinsäure oder Fumarsäure); aromatische Carbonsäuren, wie Salicilsäure; araliphatische Carbonsäuren, wie Phenylessigsäure, Phenylpropionsäure oder Zimtsäure; oder funktionalisierte Di- und Tricarbonsäuren, wie Äpfelsäure oder Zitronensäure.

Überraschenderweise tritt bei Verwendung der verschiedenartigsten Säuren zur Einstellung des für die enzymatische Umwandlung erforderlichen pH-Wertes keine Inhibierung oder Desaktivierung des Enzyms ein.

Infolgedessen können nach dem erfindungsgemäßen Verfahren die verschiedenartigsten Salze des L-Ornithins auf einfache Weise und in hoher Ausbeute hergestellt werden.

Das erfindungsgemäße Verfahren wird durch die nachfolgenden Beispiele näher erläutet. Der Ablauf der enzymatischen Umwandlung kann jeweils über die Bestimmung des gebildeten L-Ornithins beziehungsweise des verbrauchten L-Arginins durch Chromatographie verfolgt werden. Die gebildeten Salze werden durch die spezifische Drehung und durch Elementaranalyse charakterisiert.

2

Beispiel 1

130,5 g L-Arginin wurden in 800 ml $H_2O$ eingerührt und mit $\alpha$-Ketoglutarsäure auf pH = 9,5 eingestellt. Nach Zugabe von 0,042 g $MnSO_4.H_2O$ wurde der Reaktor mit $H_2O$ auf 1.000 ml aufgefüllt. Nach Zugabe von 220 mg Arginase wurde das Reaktionsgemisch 20 Stunden lang bei Raumtemperatur gerührt.

Danach wurde die Lösung mit $\alpha$-Ketoglutarsäure neutralisiert und zwecks Enzymabtrennung ultrafiltriert, im Rotationsverdampfer eingeengt und unter Kühlung mit Ethanol versetzt, wobei das Produkt auskristallisierte. Mit 166,6 g isoliertem (Di-L-Ornithin)-$\alpha$-ketoglutarat-Dihydrat betrug die Ausbeute 97 %, bezogen auf das eingesetzte L-Arginin. Das isolierte Produkt zeigte folgende analytischen Werte:

Gehalt (Titration): > 99 %

Spezifische Drehung: $[\alpha]_D^{20}$ = + 7,8° (c = 5 in $H_2O$)

| Elementaranalyse: | | | |
|---|---|---|---|
| | % C | % H | %N |
| Berechnet: | 40,3 | 7,6 | 12,55 |
| Gefunden: | 39,13 | 8,08 | 14,06 |

Trockenverlust: 9,9 %

Sulfatasche: < 0,1 %

Beispiel 2

Es wurde verfahren wie im Beispiel 1, jedoch wurden 174,3 g L-Arginin eingesetzt und es wurde L-Asparaginsäure zur Einstellung des Reaktions-pH-Wertes und zur Neutralisation des freigesetzten L-Ornithins eingesetzt.

Die Isolierausbeute an L-Ornithin-L-aspartat betrug 99 %, bezogen auf das eingesetzte L-Arginin. Das isolierte Produkt zeigte folgende analytischen Werte:

Gehalt (Titration): > 99 %

Spezifische Drehung: $[\alpha]_D^{20}$ = + 28,0° (c = 8 in 6N HCl)

| Elementaranalyse: | | | |
|---|---|---|---|
| | % C | % H | % N |
| Berechnet: | 40,71 | 7,31 | 15,83 |
| Gefunden: | 37,93 | 8,23 | 15,38 |

Trockenverlust: 0,3 %.

Beispiel 3

Es wurde verfahren wie im Beispiel 1, jedoch wurden 348,5 g L-Arginin eingesetzt und es wurde L-Glutaminsäure zur Einstellung des Reaktions-pH-Wertes und zur Neutralisation des freigesetzten L-Ornithins eingesetzt.

Die Isolierausbeute an L-Ornithin-L-glutamat betrug 96,6 %, bezogen auf das eingesetzte L Arginin. Das isolierte Produkt zeigte folgende analytischen Werte:

Gehalt (Titration): > 99 %

Spezifische Drehung: $[\alpha]_D^{20}$ = + 30,4° (c = 8 in 6N HCl)

| Elementaranalyse: | | | |
|---|---|---|---|
| | % C | % H | % N |
| Berechnet: | 42,99 | 7,52 | 15,04 |
| Gefunden: | 42,50 | 8,05 | 14,71 |

Trockenverlust: 0,7 %.

Beispiel 4

Es wurde verfahren wie im Beispiel 1, jedoch wurde L-Pyroglutaminsäure zur Einstellung des Reaktions-pH-Wertes und zur Neutralisation des freigesetzten L-Ornithins eingesetzt. Die Isolierausbeute an L-Ornithin-L-pyroglutamat-Monohydrat betrug 95,8 %, bezogen auf das eingesetzte L-Arginin. Das isolierte Produkt zeigte folgende analytischen Werte:
Gehalt (Titration): > 99 %
Spezifische Drehung: $[\alpha]_D^{20} = + 17,2°$ (c = 8 in 6N HCl)

| Elementaranalyse: | | | |
|---|---|---|---|
| | % C | % H | %N |
| Berechnet: | 43,1 | 7,52 | 15,05 |
| Gefunden: | 43,45 | 8,07 | 15,08 |

Trockenverlust: 5,4 %.

Beispiel 5

Es wurde verfahren wie im Beispiel 1, jedoch wurde $H_2SO_4$ zur Einstellung des Reaktions-pH-Wertes und zur Neutralisation des freigesetzten L-Ornithins eingesetzte. Die Isolierausbeute an (L-Ornithin)$_2$-sulfat-Monohydrat betrug 93,9 %, bezogen auf das eingesetzte L-Arginin. Das isolierte Produkt zeigte folgende analytischen Werte:
Gehalt (Titration): > 99 %
Spezifische Drehung: $[\alpha]_D^{20} = + 8,2°$ (c = 10 in $H_2O$)

| Elementaranalyse: | | | |
|---|---|---|---|
| | % C | % H | % N |
| Berechnet: | 31,57 | 7,4 | 14,73 |
| Gefunden: | 31,68 | 8,20 | 14,55 |

Trockenverlust: 4,4 %
Sulfatasche: 0,1 %.

Beispiel 6

Es wurde verfahren wie im Beispiel 1, jedoch wurde HCl zur Einstellung des Reaktions-pH-Wertes und zur Neutralisation des freigesetzten L-Ornithins eingesetzt. Die Isolierausbeute an L-Ornithin-Monohydrochlorid betrug 97,4 %, bezogen auf das eingesetzte L-Arginin. Das isolierte Produkt zeigte folgende analytischen Werte:
Gehalt (Titration): > 99 %
Spezifische Drehung: $[\alpha]_D^{20} = + 23,8°$ (c = 4 in 6N HCl)

| Elementaranalyse: | | | |
|---|---|---|---|
| | % C | % H | % N |
| Berechnet: | 35,58 | 7,71 | 16,6 |
| Gefunden: | 35,40 | 8,38 | 14,31 |

Beispiel 7

Es wurde verfahren wie im Beispiel 1, jedoch wurde Essigsäure zur Einstellung des Reaktions-pH-Wertes und zur Neutralisation des freigesetzten L-Ornithins eingesetzt. Die Isolierausbeute an L-Ornithin-acetat. betrug 95,4 %, bezogen auf das eingesetzte L-Arginin. Das isolierte Produkt zeigte folgende analytischen Werte:
Gehalt (Titration): > 99 %
Spezifische Drehung: $[\alpha]_D^{20} = +\ 10,0°$ (C = 5 in $H_2O$).

Beispiel 8

Es wurde verfahren wie im Beispiel 1, jedoch wurde D-Mandelsäure zur Einstellung des Reaktions-pH-Wertes und zur Neutralisation des freigesetzten L-Ornithins eingesetzt. Die Isolierausbeute an L-Ornithin-D-mandelat-Dihydrat betrug 94,2 %, bezogen auf das eingesetzte L-Arginin. Das isolierte Produkt zeigte folgende analytischen Werte:
Gehalt (Titration): > 99 %
Spezifische Drehung: $[\alpha]_D^{20} = -\ 52,7°$ (c = 2 in $H_2O$)

| Elementaranalyse: | | | |
|---|---|---|---|
| | % C | % H | % N |
| Berechnet: | 48,75 | 7,5 | 8,75 |
| Gefunden: | 49,50 | 7,7 | 8,85 |

Trockenverlust: 9,4 %.

Beispiel 9

Es wurde verfahren wie im Beispiel 1, jedoch wurde Phosphorsäure zur Einstellung des Reaktions-pH-Wertes und zur Neutralisation des freigesetzten L-Ornithins eingesetzt. Die Isolierausbeute an (L-Ornithin)$_3$-phosphat-Monohydrat betrug 93,1 %, bezogen auf das eingesetzte L-Arginin. Das isolierte Produkt zeigte folgende analytischen Werte:
Gehalt (Titration): > 99 %
Spezifische Drehung: $[\alpha]_D^{20} = +\ 20,4°$ (c = 8 in 6N HCl)

| Elementaranalyse: | | | |
|---|---|---|---|
| | % C | % H | % N |
| Berechnet: | 35,1 | 8,0 | 16,4 |
| Gefunden: | 30,19 | 7,47 | 14,7 |

Trockenverlust: 3,7 %.

Beispiel 10

Es wurde verfahren wie im Beispiel 1, jedoch wurde Glutathion in Form des Disulfids (GSSG) zur Einstellung des Reaktions-pH-Wertes und zur Neutralisation des freigesetzten L-Ornithins eingesetzt. Die Isolierausbeute an (L-Ornithin)$_2$-glutathiondisulfid-Dihydrat betrug 97,7 %, bezogen auf das eingesetzte L-Arginin. Das isolierte Produkt zeigte folgende analytischen Werte:
Gehalt (Titration): > 99 %
Spezifische Drehung: $[\alpha]_D^{23} = -\ 70,3°$ (c = 1,9 in $H_2O$)

| Elementaranalyse: | | | |
|---|---|---|---|
| | % C | % H | % N |
| Berechnet: | 38,4 | 6,14 | 14,34 |
| Gefunden: | 37,7 | 7,32 | 14,82 |

Trockenverlust: 5,4 %.

**Patentansprüche**

1.  Verfahren zur Herstellung von Salzen des L-Ornithins durch enzymatische Umwandlung von Arginin in L-Ornithin in Gegenwart des Enzyms L-Arginase in einem wäßrigen Medium und anschließende Salzbildung,
    dadurch gekennzeichnet,
    daß man den für die enzymatische Umwandlung erforderlichen pH-Wert im Bereich von 8,0 bis 10,0 mit der Säure einstellt, deren Salz hergestellt werden soll, nach beendeter enzymatischer Umwandlung das Reaktionsgemisch mit der gleichen Säure auf einen pH-Wert im Bereich von 6,5 bis 7,0 einstellt und das gebildete Salz direkt aus dem Reaktionsgemisch isoliert.

2.  Verfahren nach Anspruch 1, dadurch gekennzeichnet, daß man vor der Isolierung des gebildeten Salzes das Enzym durch Ultrafiltration aus dem Reaktionsgemisch abtrennt.

**Claims**

1.  Process for the preparation of salts of L-ornithine by enzymic conversion of arginine into L-ornithine in the presence of the enzyme L-arginase in an aqueous medium and subsequent salt formation, characterised in that
    the pH value required for the enzymic conversion is adjusted to within the range of from 8.0 to 10.0 by means of the acid the salt whereof is to be prepared, after enzymic conversion is complete, the reaction mixture is adjusted, by means of the identical acid, to a pH value in the range of from 6.5 to 7.0, and the salt formed is isolated directly from the reaction mixture.

2.  Process according to claim 1, characterised in that prior to the isolation of the salt formed, the enzyme is separated from the reaction mixture by ultrafiltration.

**Revendications**

1.  Procédé pour la préparation de sels de L-ornithine par transformation enzymatique d'arginine en L-ornithine en présence de l'enzyme L-arginase dans un milieu aqueux suivie d'une formation de sel, caractérisé en ce qu'on règle le pH nécessaire pour la transformation enzymatique dans la plage comprise entre 8,0 et 10,0 au moyen de l'acide dont on veut fabriquer le sel, en ce qu'on règle, après la transformation enzymatique, le pH du mélange réactionnel au moyen du même acide dans la plage comprise entre 6,5 et 7,0 et en ce qu'on isole directement le sel formé à partir du mélange réactionnel.

2.  Procédé selon la revendication 1, caractérisé en ce qu'on sépare l'enzyme par ultrafiltration du mélange réactionnel avant d'isoler le sel formé.